# EUROPEAN PATENT SPECIFICATION

(11) **EP 3 989 894 B1**
(45) Date of publication and mention of the grant of the patent: **29.07.2026**
(21) Application number: 20744128.8
(22) Date of filing: 25.06.2020
(51) Int. Cl.: A61F 7/00

(54) **CLINICAL GARMENT WITH A CONVECTIVE DEVICE**
KLINISCHES KLEIDUNGSSTÜCK MIT EINER KONVEKTIONSVORRICHTUNG
VÊTEMENT CLINIQUE AYANT UN DISPOSITIF DE CONVECTION

(30) Priority: 26.06.2019 US 201962866686 P
(43) Date of publication of application: 04.05.2022
(73) Proprietor: Solventum Intellectual Properties Company, Eagan, MN 55121 (US)
(72) Inventor: MCGREGOR, Andrew J., Saint Paul, Minnesota 55133-3427 (US); STARK, John R., Saint Paul, Minnesota 55133-3427 (US); THIELEN, James A., Saint Paul, Minnesota 55133-3427 (US); LINDSAY, Jenna L., Saint Paul, Minnesota 55133-3427 (US); DORAN, Daniel P., Saint Paul, Minnesota 55133-3427 (US); RUE, Amanda M., Saint Paul, Minnesota 55133-3427 (US); STANAWAY, Benjamin C., Saint Paul, Minnesota 55133-3427 (US)
(74) Representative: Vossius & Partner Patentanwälte Rechtsanwälte mbB
(86) International application number: PCT/IB2020/056040
(87) International publication number: WO 2020/261186

(56) References cited:
- EP-A1- 1 937 196
- WO-A1-2014/092874
- WO-A2-2006/086587
- US-B1- 6 245 096
- US-B2- 7 862 599

## Description

### BACKGROUND

A warming device for perioperative use includes a clinical garment with one or more pneumatic convective devices supported on the inside of the garment. Pneumatic convective devices that transfer heat to a human body are known. For example, there are devices that receive a stream of pressurized, warmed air, inflate in response to the pressurized air, distribute the warmed air within a pneumatic structure, and emit the warmed air onto a body to accomplish such objectives as increasing comfort, reducing shivering, and treating or preventing hypothermia. These devices are typically called "convective thermal blankets" or "covers"; for convenience, in this discussion and the following specification, they shall be called, simply, "thermal blankets." 3M makes and sells such devices under the BAIR HUGGER trade designation. One such device is the Model 622 Upper Body Blanket. Use of the term "convective" to denote the transfer of heat between the warming device and a body refers to the principal mode of heat transfer, it being understood that heat may at the same time be transferred between a warming device and a body by conduction and radiation, although not to the degree of convection.

In some embodiments, a clinical garment such as a robe or gown to receive a pneumatic convective device in order to warm a person wearing the garment in a clinical setting for comfort and mobility of the person. There is a need to further adapt such a combination for use perioperatively.

The effectiveness of therapeutic warming depends upon delivery of enough heat to a patient's body to raise the patient's core body temperature to, or maintain it within, a narrow range, typically near 37°C. This range is called "normothermic" and a body with a core temperature in this range is at "normothermia." Hypothermia occurs when the core body temperature falls below 36°C; mild hypothermia occurs when core body temperature is in the range of 34°C to 36°C. Therefore, "perioperative therapeutic warming" is warming therapy capable of being delivered during one or more of the perioperative periods for the prevention or treatment of hypothermia.

Therapeutic warming is contrasted with "comfort warming" which is intended to maintain or enhance a patient's sense of "thermal comfort". Of course, therapeutic warming may also comfort a patient by alleviating shivering or a feeling of being cold, but this is a secondary or ancillary effect. Thermal comfort is a subjective notion; however, the environmental conditions necessary to produce a sense of thermal comfort in a population of human beings are known and well tabulated. For example, Fanger (Thermal Comfort: Analysis and Applications of Environmental Engineering. Danish Technical press, Copenhagen, 1970) defines thermal comfort as "that condition of mind which expresses satisfaction with the thermal environment." Even when a patient is normothermic, less than ideal environmental conditions can result in acute feelings of discomfort. Under normothermic conditions, thermal comfort is largely determined with reference to skin temperature, not core body temperature.

Comfort warming is warming applied to a patient to alleviate the patient's sense of thermal discomfort. Therapeutic warming may be indicated during any one or more of the perioperative periods. For example, for a short operation in a surgery with no warming equipment available, a person may be warmed preoperatively in a preparation area to raise mean body temperature to a level higher than normal in order to store enough thermal energy to maintain normothermia, without heating, intraoperatively. After surgery, it may be necessary to apply therapeutic warming in a recovery area to raise the core temperature to normothermia and maintain it there for a period of time while anesthesia wears off. Alternatively, for a long surgery in an arena with heating equipment available, a person may be warmed for comfort before surgery and warmed therapeutically during and after surgery. Both therapeutic warming and comfort warming may be provided by convective devices such as convective thermal blankets that receive and distribute warmed, pressurized air and then expel the distributed air through one or more surfaces toward a patient in order to prevent or treat hypothermia in the patient.

When delivered by convective devices, therapeutic warming is distinguished from comfort warming by intended effects and by the parameters of heat delivery that produce those effects. In this regard, a convective warming system typically includes a source of warmed pressurized air (also called a heater/blower unit, a forced air warming unit, a heater unit, etc.), a convective device such as a thermal blanket (which is, typically, inflatable), and a flexible conduit or air hose connecting the heater/blower unit with the thermal blanket. Use of such a system for a particular type of warming requires delivery of warmed air through a thermal blanket at parametric values that achieve a particular objective. The conditions by which a convective device such as a thermal blanket produces thermal comfort in normothermic individuals at steady state are significantly different from those necessary to treat hypothermia. Typically the conditions for thermal comfort are met in a system with a relatively low capacity heater/blower unit, while those in a therapeutic warming system are achieved with a relatively high capacity heater/blower unit. The different capacities have led to use of air hoses with different capacities, with those delivering air flow for thermal comfort typically having smaller diameters than those serving a therapeutic warming requirement. The result is a divergence of designs leading to installation of different air delivery infrastructures for therapeutic and comfort warming.

WO 2014/092874 A1 relates to a warming device including a clinical garment with a flexible inflatable convective apparatus integrated with or supported on a surface of the garment for warming one or more limbs when the apparatus is inflated with warmed air. The convective apparatus includes a central member, one or more peripheral diffusers, and one or more flexible ducts. In some cases, each duct connects a diffuser with the central member. One or more inlet ports are provided in the convective apparatus to receive the end of an air hose. The central member is inflatable in response to a flow of air through an inlet port. A peripheral diffuser is positioned on the clinical garment so as to be deployable to or near to an extremity such as a limb. When a peripheral diffuser is deployed while the central member is inflated with warmed air, the warmed air flows from the central member, through a duct to the peripheral diffuser and is emitted through an air-permeable wall of the diffuser to warm the extremity.

EP 1 937 196 A1 relates to a multifunction warming device for perioperative use including a clinical garment and at least one convective warming apparatus supported on an inside surface of the clinical garment. One convective apparatus is disposed transversely in an upper portion of the clinical garment, between two sleeves of the clinical garment. A second convective apparatus with separately inflatable sections, each for enabling a particular mode of warming, may be disposed longitudinally in a lower portion of the clinical garment. In either case, an attachment mechanism is provided adjacent a convective apparatus for adhesively attaching to a person.

WO 2006/086587 A2 relates to a warming device for perioperative use including a clinical garment and a convective thermal blanket supported on an inside surface of the clinical garment. A mechanism may be provided to releasably attach the thermal blanket to the clinical garment.

US 7 862 599 B2 relates to a convective warming device including a clinical garment and at least one inflatable convective apparatus supported on an inside surface of the garment which is provided with a drape. When the convective warming device is used to warm a person, the drape is deployed over skin of the person not covered by the clinical garment. The drape protects the covered skin from thermal injury by an air hose used to conduct heated pressurized air to the inflatable convective apparatus.

US 6 245 096 B1 relates to a thermal cover member for delivering a fluid to a patient's body, such as heated or cooled air, including a hollow housing member having an inlet port for receiving the pressurized fluid. A plurality of cells are positioned about the housing member to create fluidic spaces for applying the fluid to the body of the patient. Each cell has a non-inflatable canopy with a continuous perimeter sealingly connected to the housing member with the housing member having a plurality of exit ports communicating with each cell to provide an egress of fluid. The fluidic spaces within a cell can be dimensioned to accommodate anatomical configurations of the patient's body and a thermo-chromatic indicator can be provided on the cover member to indicate a temperature of the corresponding fluidic space. The canopy can be transparent to permit viewing of the patient's body.

### SUMMARY

The invention is defined by the features of the independent claims. Preferred embodiments are defined in the dependent claims.

While some clinical garments have built in pneumatic convective devices that fold out, none of the solutions are configured to provide warming to the legs to be used with a pre-warming solution. Further, such devices may be used for comfort warming as opposed to therapeutic warming due to the pneumatic surface area of the pneumatic convective device.

Aspects of the present disclosure relate to a warming device that includes a clinical garment and a first pneumatic convective device. The clinical garment includes an inner surface for facing the patient and having an inner surface area. The clinical garment has a hemline and forms a longitudinal axis. The first pneumatic convective device can have an air-permeable layer having a pneumatic surface area, the pneumatic surface area is at least 25% of the inner surface area in a deployed state. The warming device can include a first inlet for admitting a stream of pressurized, warmed air into the first pneumatic convective device. The second end of the first pneumatic convective device extends past the hemline when in the deployed state, and, when the first pneumatic convective device is in a folded state, the second end extends no greater than 7.6 cm (3 inches) past the hemline.

Additional aspects also relate to a system and a method (not part of the claimed invention) of warming a patient using the warming device.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates a perspective drawing showing assembly of a warming device constituted of a clinical garment with a hemline and a pneumatic convective device disposed longitudinally along the inside of the garment in accordance with one embodiment.
FIG. 2 illustrates a cross-sectional view of a folded state the warming device of FIG. 1 in accordance with one embodiment.
FIG. 3 illustrates a perspective view of the folded state of the warming device of FIG. 1 in accordance with one embodiment.
FIG. 4 illustrates another perspective view of a partially deployed state of the warming device of FIG. 1 in accordance with one embodiment.
FIG. 5 illustrates another perspective view of a partially deployed state of the warming device of FIG. 1 in accordance with one embodiment.
FIG. 6 illustrates a perspective view of the deployed state of the warming device of FIG. 1 in accordance with one embodiment.
FIG. 7 illustrates a perspective view of the tab element of the warming device of FIG. 1 in accordance with one embodiment.
FIG. 8 illustrates a perspective view of a warming device in accordance with one embodiment.
FIG. 9 illustrates a cross-sectional view of the warming device of FIG. 8 in accordance with one embodiment.
FIG. 10 illustrates a flow chart of a method of prewarming a patient in accordance with one embodiment.

### DETAILED DESCRIPTION

"Clinical garment" refers to a garment that is typically used to temporarily clothe a patient in a clinical setting. Such garments include hospital gowns, robes, bibs and other equivalents. The clinical setting may be a medical or dental office or clinic, a hospital, or any facility or institution that provides medical or dental treatment to patients.

"Pneumatic convective device" refers to refers to a device that receives and distributes at least one stream of warmed pressurized air in a structure for being disposed on, adjacent, or next to the core and/or the limbs of a body. When pressurized with warmed air, the pneumatic convective device emits warmed air through one or more of its surfaces. The pneumatic convective device may be releasably attached to the inside surface of the garment. In one aspect, a pneumatic convective device for perioperative use may be worn on a person where it receives a stream of warmed pressurized air, distributes the pressurized air within the attached thermal blanket, and emits the air through one or more surfaces of the thermal blanket to warm the person's body. In another aspect, the pneumatic convective device may be adapted for therapeutic warming during surgery. In yet another aspect, the pneumatic convective device may be adapted for therapeutic warming by detaching the thermal blanket from the clinical garment for deployment intraoperatively. In yet another aspect, the pneumatic convective device may be reattached to the clinical garment for further use on the same person during the postoperative period.

"Pneumatic surface area" refers to surface area of the inflated portion of the pneumatic convective device.

"Pneumatic edge portion" refers to the edge of the pneumatic cavity which is different from the edge of the pneumatic convective device as the edge of the pneumatic convective device include unsecured sheets.

"Sheet" refers to an extensive unbroken surface area of a material. A sheet is generally continuous and has a mostly uniform thickness.

"Perioperative" refers to "around the time of operation" as defined in the PDR Medical Dictionary, Second Edition, (Medical Economics Company, 2000). The perioperative period is characterized by a sequence including the time preceding an operation when a patient is being prepared for surgery ("the preoperative period"), followed by the time spent in surgery ("the intraoperative period"), and by the time following an operation when the patient is closely monitored for complications while recovering from the effects of anesthesia ("the postoperative period").

"Inflatable" refers to a structure that is flaccid when not in use and that tautens when receiving a stream of pressurized air.

"Clinician" refers to medical personnel having direct contact with and responsibility for patients. Can include doctors, physician assistants, nurse practitioners, nurses, nurse assistants, home aides.

"Prewarming" refers to warming during the period before anesthesia or before an incision is made in the patient during a surgical procedure

"Fastening device" refers to a device or technique that fastens two sheets together.

"Recloseable fastener" refers to a type of fastening device to allow repositioning of an article from a first application surface to a second application surface without damaging either the article or the first or second application surface.

"Fixed fastener" refers to a fastening device that will damage the underlying material if separated.

Aspects of the present disclosure relate to a warming device that includes a clinical garment and a pneumatic convective device disposed thereon. An end of the pneumatic convective device is configured to extend past the hemline of the clinical garment in a deployed state.

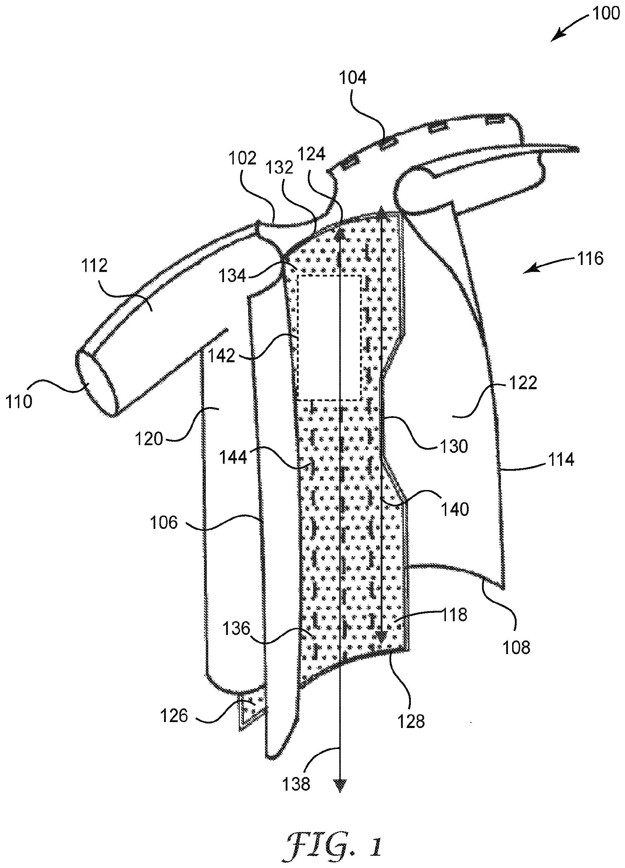

FIG. 1. illustrates another aspect of the warming device 100 for perioperative use. In this case, the pneumatic convective device 118 is supported on the inner surface 122, longitudinally, that is to say, in alignment with the longitudinal axis 138 of the clinical garment 116. In at least one embodiment, the pneumatic convective device 118 can be releaseably attached to the inner surface 122. The clinical garment 116 also includes an outer surface 120, opposite the inner surface 122.

In at least one embodiment, the clinical garment 116 can be made from a woven cloth, such as cotton, or a non-woven such as spunbond-meltblown-spunbond material (SMS) or a synthetic sheet material. Examples of non-woven material include any one or more of polyester, cotton, rayon, polypropylene, and wood pulp. Examples of synthetic sheet material include polypropylene, polyesters, and polyurethanes. In at least one embodiment, the clinical garment 116 can be treated the synthetic sheet material to make a portion of the clinical garment 116 air-impermeable. In at least one embodiment, the clinical garment 116 is a disposable gown that is configured to be single-use by a patient before the clinical garment 116 is disposed as medical waste.

The clinical garment 116 has rear slit 106 and rear slit 114 which are opposing each other. The rear slit 106 and rear slit 114 can extend from the neck opening 102 to a hemline 108. In at least one embodiment, the distance from the neck opening 102 to the hemline 108 can be at least 61 cm (24 inches), at least 76 cm (30 inches), or at least 91 cm (36 inches).

To attach the clinical garment 116 to a patient, there is a fastening device 104 provided to provide for ease in securing the clinical garment 116 to the patient as well as allowing for ease in adjusting the size of the clinical garment 116 to accommodate various different size wearers. For example, additional fastening devices can be positioned along opposing sides of the rear slit 106 and rear slit 114 that can be brought together and fastened to hold the clinical garment 116 to the patient. Another fastening device is a plurality of strings positioned along opposing sides of the rear slit 106 and rear slit 114 that can be tied together to hold the clinical garment 116 to the patient. Other fastening devices include ultrasonic or thermal welding, snaps, repositionable adhesive, hook and loop elements, double-sided adhesive, snaps, rivets, and any and all equivalents thereof.

In some embodiments, the clinical garment may include sleeves that are sized and positioned for receiving a patient's arms. For example, the sleeves 112 can have slits that run the entire length on the shoulder. This allows access to the upper body of the patient and allows for opening and closing of the slits in an adjustable fashion using fastening device 104. The design also facilitates the manufacturing of the clinical garment 116 in one piece. The slit can extend from the neck opening 102 to the cuff 110.

The sleeves 112 can have a diameter. In at least one embodiment, the diameter can vary from the armhole end and the cuff 110 near the hands of a patient. In at least one embodiment, the diameter can be 15 - 38 cm (6 - 15 inches), 20 - 38 cm (8 - 15 inches), 15 - 38 cm (10 - 15) inches, or 25 - 36 cm (10 - 14 inches) near the cuff 110.

The warming device 100 includes a pneumatic convective device 118 that is substantially centered (along the width dimension) in the inside of the clinical garment 116. In at least one embodiment, the pneumatic convective device 118 is attached to the inner surface 122 such that end 136 (e.g., including pneumatic edge portion 128) extends past the hemline 108 when in a deployed state and end 136 extends no greater than 13 cm (5 inches), no greater than 10 cm (4 inches), no greater than 7.6 cm (3 inches), no greater than 5.1 cm (2 inches), no greater than 2.5 cm (1 inch), or does not extend past the hemline 108 when in a folded state.

In at least one embodiment, the pneumatic convective device 118 can be a separate forced air warming blanket such as an upper body blanket that is commercially available as the model 622 under the trade designation Bair Hugger.

For example, the pneumatic convective device 118 includes two generally rectangular sheets of material (e.g., sheet 126 and sheet 202) that are sealed together continuously at their perimeter 124 and intermittently at multiple locations within their peripheries (which is referred to as a staked seal 144 having a diameter less than 5.1 cm (2 inches)).

As shown, the pneumatic convective device 118 can be formed from two sheets having the same generally quadrilateral shape, with an optional U-shaped indentation along side pneumatic edge portion 130. In other embodiments, the pneumatic convective device 118 can be a single sheet of material that is bound to the clinical garment 116 itself (as illustrated herein). In at least one embodiment, the pneumatic convective device 118 can include at least one side pneumatic edge portion 130, pneumatic edge portion 132, and pneumatic edge portion 128. The pneumatic convective device 118 can also have an end 134 and end 136. In at least one embodiment, end 134 includes pneumatic edge portion 132 and end 136 includes pneumatic edge portion 128. As described herein, the term end can refer to a general region of the pneumatic convective device 118 whereas the term edge portion can refer specifically to the edge of pneumatic convective device 118. In at least one embodiment, the edge portion and end can be used interchangeably.

In at least one embodiment, the clinical garment 116 can have a longitudinal axis 138. The pneumatic convective device 118 can be disposed along the longitudinal axis 138 such that end 134 is aligned with the neck opening 102 and the end 136 is aligned with the leg extremity of a patient when warming device 100 is worn.

The pneumatic convective device 118 can have a longitudinal dimension 140 that is dimensioned to cover the torso of a patient and a portion of the leg extremities. In at least one embodiment, the longitudinal dimension 140 is at least 150 cm, at least 160 cm, at least 170 cm, at least 180 cm, at least 190 cm. In at least one embodiment, the longitudinal dimension 140 can be from 190 cm to 210 cm.

In at least one embodiment, the warming device 100 can also include a second pneumatic convective device 142. The pneumatic convective device 142 can be disposed on the inner surface 122 or on the pneumatic convective device 118. For illustrative purposes, pneumatic convective device 142 is disposed on the inner surface 122. For example, pneumatic convective device 142 can be releasably attached to the inner surface 122 of the clinical garment 116. The pneumatic convective device 142 can be formed from a single sheet attached to the clinical garment 116 or can be constructed with two sheets. The pneumatic convective device 118 can be releasably attached to the surface of pneumatic convective device 142.

In at least one embodiment, a plurality of islands of fastening devices such as pressure sensitive adhesive material, hook and loop material, snaps, or other equivalent elements may be provided on the pneumatic convective device 142. The islands may be arranged in a pattern that lies on an outline of the periphery of the pneumatic convective device 142. Corresponding elements may be provided on the posterior surface of the pneumatic convective device 142 which faces the inner surface 122. A second opening in the clinical garment 116 can provide access by which an air hose can connect to an inlet port of the pneumatic convective device 142. Warmed, pressurized air flowing into the pneumatic convective device 142 exits through apertures in the surface.

In at least one embodiment, the warmed air is provided through the pneumatic convective device 142 and/or pneumatic convective device 118. During a preoperative period, the patient can be therapeutically heated or comfort heated, but preferably therapeutically heated to modify the patient's core temperature. In the preoperative period, the pneumatic convective device 118 can be converted from the folded state to a partially deployed state or a deployed state.

When the patient transitions to surgery, the pneumatic convective device 118 can be detached from the warming device 100. Operatively, the pneumatic convective device 118 may be used alone for therapeutic heating; alternatively, the pneumatic convective device 118 or the pneumatic convective device 142 may be reattached to the clinical garment 116 for therapeutic or comfort heating.

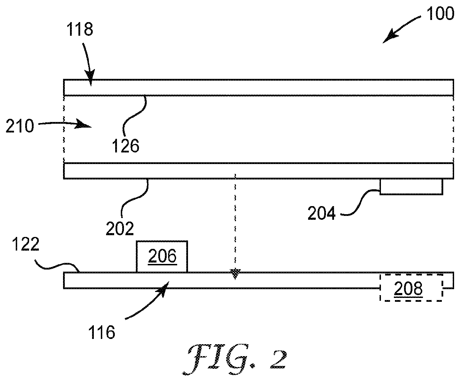

FIG. 2 illustrates a side cross-sectional view of warming device 100. As described herein, the pneumatic convective device 118 can be attached to the inner surface 122 of clinical garment 116 via fastening device 206. In at least one embodiment, the fastening device 206 can be permanent (fixed) or recloseable. For example, a fixed fastener/fastening device 206 can include two-sided adhesive, sewing, heat, ultrasonic welding, rivets, and any and all equivalents thereof. A recloseable fastener can include hook and loop, snaps, repositionable adhesive, or combinations thereof. Preferably, the fastening device 206 can be recloseable.

To attach the pneumatic convective device 118 to the clinical garment 116, the fastening device 206 may be disposed between the pneumatic convective device 118 and the inner surface 122 . The fastening of the pneumatic convective device 118 (i.e., sheet 202) with the inner surface 122 can enable the pneumatic convective device 118 to be mounted to, received on, supported by or otherwise combined with the clinical garment 116, with the pneumatic edge portion 132 adjacent the edge of neck opening 102 of the clinical garment 116 which receives the neck of a user, sheet 126 facing the wearer of the clinical garment 116.

In at least one embodiment, the pneumatic convective device 118 can be releasably attached to the clinical garment 116. For example, the pneumatic convective device 118 can have a strip of releasable adhesive that attaches to the clinical garment 116 but is releasable when a shear force is applied. In another example, the pneumatic convective device 118 can include a perforation proximate a permanent adhesive (i.e., fixed fastener) such that a user can tear along the perforation cleanly to remove the pneumatic convective device 118. In another example, the pneumatic convective device 118 can include hook and loop fastening devices.

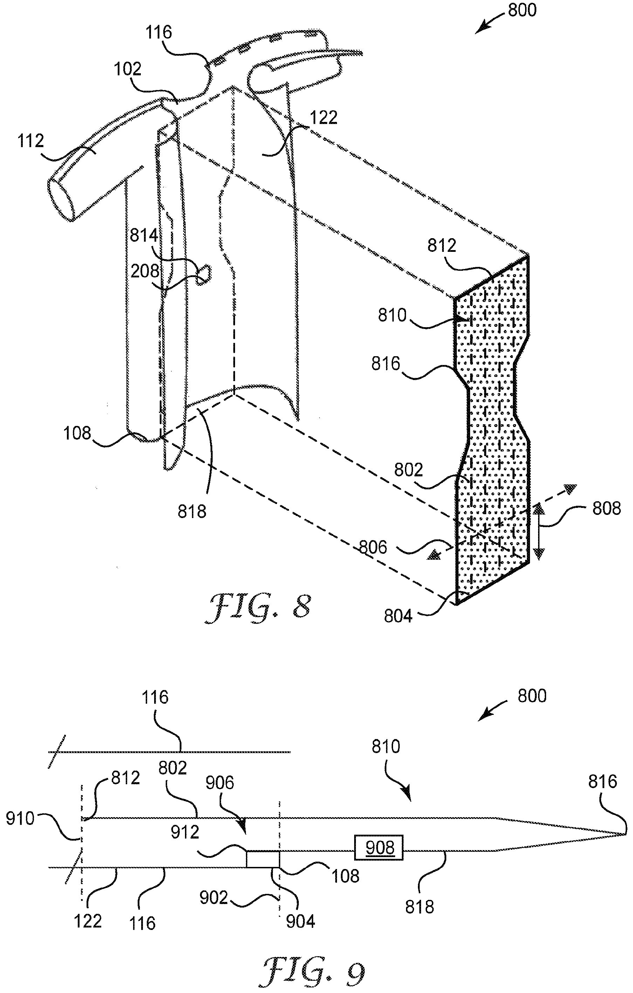

As shown, the pneumatic convective device 118 may be a separate piece which is attached to the garment, or it may be formed integrally with the garment as described above in connection with FIG. 8 and FIG. 9.

In at least one embodiment, the pneumatic convective device 118 can be formed from a sheet 126 and sheet 202 as described herein. At least one of the sheets is permeable to air. In this example, the sheet 126 is air permeable, although this is not intended to so limit the scope of the invention. The permeability of the sheet 126 may be provided by characteristics of the material from which it is formed; alternatively, holes or apertures may be formed in it during the process which joins the sheets. Or, permeability of the sheet 126 may result from the characteristics of its formative material and from formed apertures. The pneumatic convective device 118 can be sealed along a perimeter 124 to form a cavity 210. The seal is preferably air-tight such that air is forced through a portion of sheet 126. For example, warmed, pressurized air flowing into the pneumatic convective device 118 exits through apertures in the surface. In at least one embodiment, the sheet 202 or sheet 126 can have an inlet 204 for receiving an air hose from a warming unit. In at least one embodiment, the inlet 204 can be formed from a portion of the sheet therein (i.e., by a connection or puncture of an air nozzle from the air hose). The inlet 204 can further secure the air nozzle such that the air nozzle does not fall out. In at least one embodiment, the inlet 204 can include a hose card that is affixed to the sheet. For example, the hose card can be generally rectangular and have a hole formed therein. The hole can be dimensioned to receive the air nozzle tip.

In at least one embodiment, the clinical garment 116 can have an optional opening 208 to access the inlet 204. The opening 208 in the clinical garment 116 provides access by which an air hose can connect to the inlet 204 of the pneumatic convective device 118.

In at least one embodiment, the inlet 204 can be aligned with the opening 208 when pneumatic convective device 118 is in a folded state but can extend beyond a hemline of the clinical garment 116 when the pneumatic convective device 118 is in a partially deployed state or deployed state.

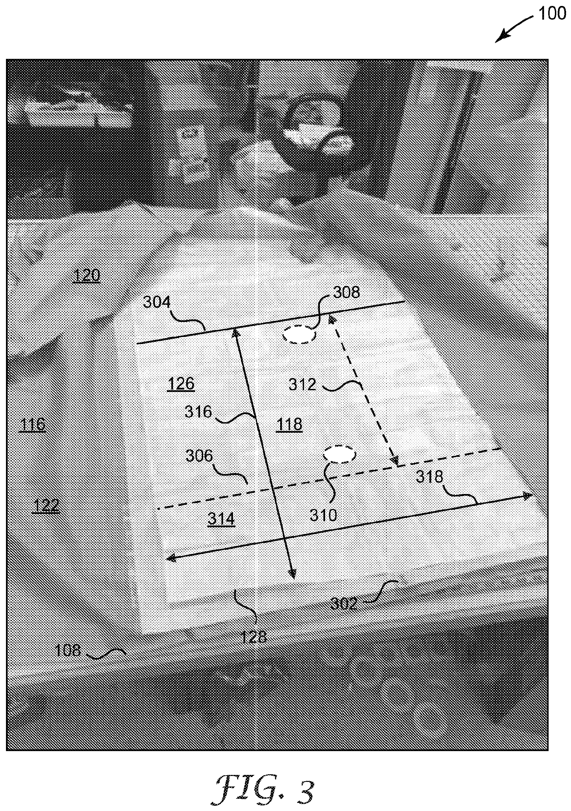

FIG. 3 illustrates the warming device 100 having a pneumatic convective device 118 and a clinical garment 116. The clinical garment 116 can have an outer surface 120 and inner surface 122. The pneumatic convective device 118 can be attached to the inner surface 122 as described further herein. FIG. 3 shows the pneumatic convective device 118 in a folded state. In the folded state, the pneumatic convective device 118 can be folded such that the pneumatic edge portion 128 or end 136 extends no greater than 7.6 cm (3 inches) or does not extend past the hemline 108. In at least one embodiment, the pneumatic convective device 118 can also include a tab element 302 protruding from the pneumatic edge portion 128. When in the folded state, the tab element 302 can protrude slightly from the hemline 108 such that a clinician can easily change the pneumatic convective device 118 from a folded state to a partially deployed state or deployed state.

The fold can be temporary and configured to extend the pneumatic convective device 118 onto legs of a patient when in a deployed state. In at least one embodiment, the fold can be also allow the pneumatic convective device 118 to be used to warm an area of the patient in a folded state that is less than an area of the patient when the pneumatic convective device 118 is in a deployed state or partially deployed state. As shown in warming device 100, the pneumatic convective device 118 is shown with a z-fold in a folded state.

When in the folded state, the pneumatic convective device 118 has a plurality of fold sections. Each fold section has a fold edge portion which is formed from creasing the pneumatic convective device 118. Each fold section can further have one or more fold dimensions. For example, fold section 314 (which is closest to the patient), can have a fold dimension 316 along a longitudinal axis and width dimension 318. The pneumatic convective device 118 can also have another fold section (not shown in FIG. 3) defined by fold edge portion 306 and fold dimension 312. According to the invention, the different fold sections are attached to other fold sections using fastening devices, e.g., fastening device 308 and fastening device 310. As shown, the fastening devices are releasable and are disposed proximate to a fold edge portion.

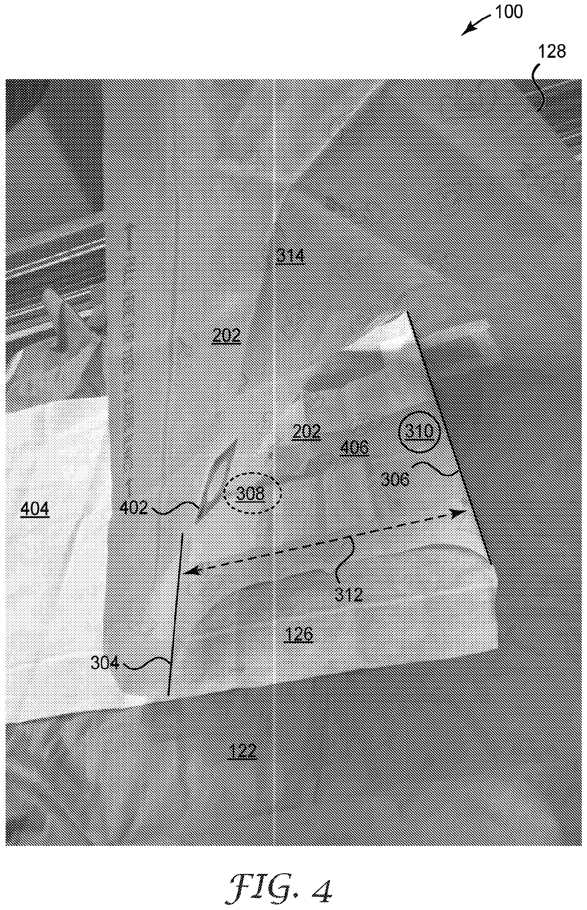

FIG. 4 illustrates a pneumatic convective device 118 in a partially deployed state. For example, fold section 314 is lifted to show fold edge portion 304 and fold edge portion 306. In at least one embodiment, the fold section 406 can be defined by the fold edge portion 304 and fold edge portion 306. A fold dimension 312 can exist between fold edge portion 304 and fold edge portion 306 along the longitudinal axis.

The fold section 314 can also have a hose card 402 disposed on sheet 202 to form an inlet. In at least one embodiment, the pneumatic convective device 118 can be secured in the folded state by fastening device 308 and fastening device 310. The fastening device 310 can be positioned to be approximately centered (in the width dimension and/or along a longitudinal axis) on the opposing fold section. For example, fastening device can be positioned on the fold section 406 such that fastening device 310 aligns with a non-edge portion of fold section 314.

In at least one embodiment, fold section 404 is defined from an edge portion (proximate the neck opening) to fold edge portion 306. When inflated in the folded state, the fold section 404 can emit pressurized, warmed air to a portion of fold section 404 while the fold edge portion 306 prevents or impedes airflow to fold section 406 or fold section 314.

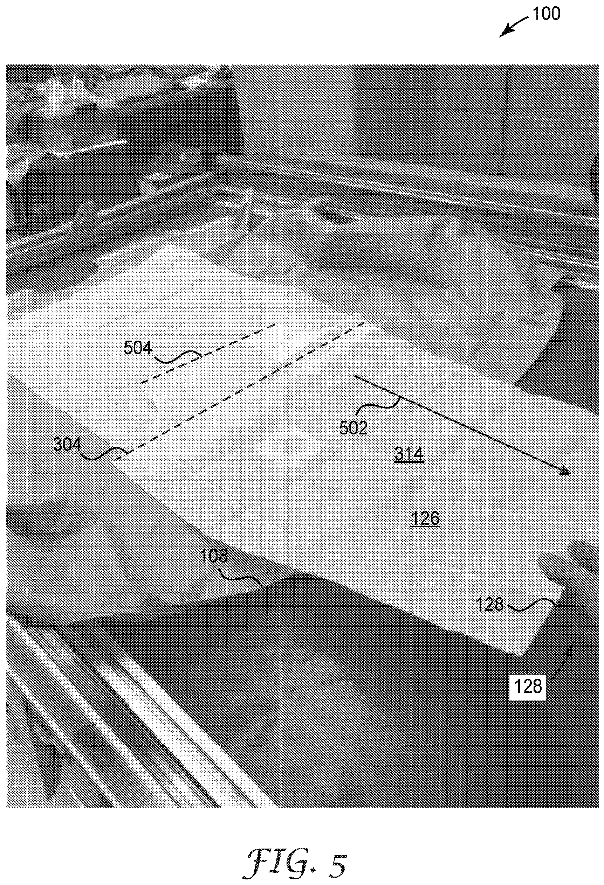

FIG. 5 illustrates the warming device 100 transitioning from a folded state to a partially deployed state. A user can exert a (lateral) pull force 502 on the fold section 314 to change the position of the fold edge portion 304 to the positioning of fold edge portion 504. In at least one embodiment, the fold edge portion can be related to the degree of pull force 502. As the position of the fold edge portion 304 is changed, the dimensions of the surface area and the warmed air provided to a patient is changed. As shown, the pneumatic edge portion 128 can extend beyond hemline 108.

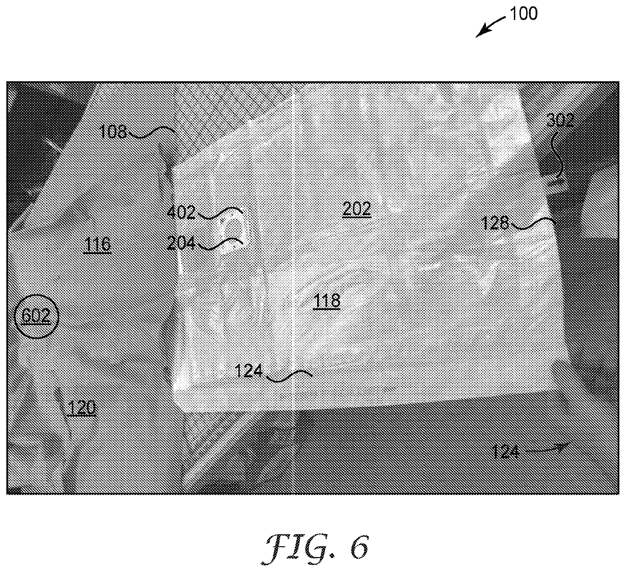

FIG. 6 shows the warming device 100 and the pneumatic convective device 118 in a deployed state. In the deployed state, the pneumatic edge portion 128 extends beyond the hemline 108 and is configured to warm a portion of the legs of the patient. In at least one embodiment, the inlet 204 and hose card 402 can extend past the hemline 108 of the clinical garment 116. In use, a clinician can connect a convective warming unit to the inlet 204 and inflate the pneumatic convective device 118 to provide warmed air to the patient. In at least one embodiment, the patient can be positioned in a supine position prior to a surgical procedure.

In at least one embodiment, the pneumatic convective device 118 is configured to be secured to the front side of the clinical garment 116 (which corresponds to the front side of a patient). For example, a fastening device 602 can be present on the outer surface 120 and configured to secure a portion of pneumatic convective device 118 (e.g., sheet 202) that is proximate to the pneumatic edge portion 128. A portion of the pneumatic convective device 118 is folded over hemline 108 and secured to the fastening device 602. Thus, the pneumatic convective device 118 is positioned in a folded up state so that the patient does not trip when ambulatory. In at least one embodiment, the fastening device 602 is a recloseable fastener.

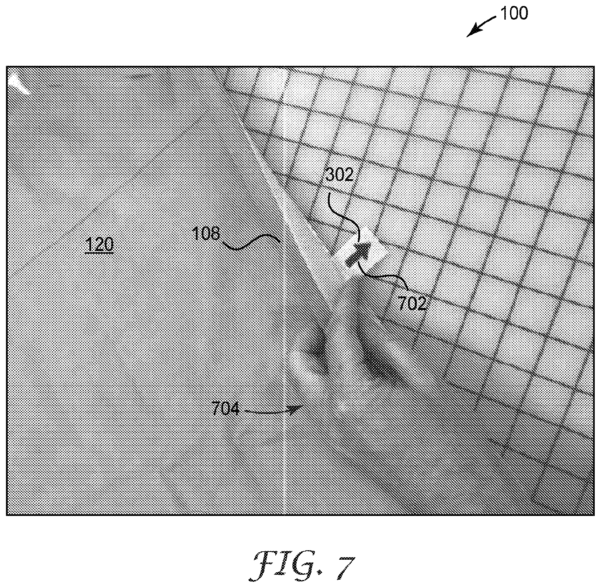

FIG. 7 illustrates a closer view of the tab element 302. In at least one embodiment, the tab element 302 can be positioned proximate the hemline 108. As shown, as the clinical garment 116 is lifted at the hemline 108, the tab element 302 is exposed. The tab element 302 can also include a visual indication 702 to indicate the direction to apply a pull force. FIG. 7 also illustrates a clinician 704 which can be a part of the overall warming system including the warming device 100, hose, warming unit, and/or patient. Examples of the warming unit are commercially available under the trade designation Bair Hugger Model 675, 775, or 875.

FIG. 8 illustrates a warming device 800 having a pneumatic convective device 810 and clinical garment 116. The clinical garment 116 can be the same as in warming device 100. For example, the clinical garment 116 can include a neck opening 102, sleeves 112, hemline 108, and opening 208. The opening 208 and inlet 814 can lead into a cavity 906 formed by the pneumatic convective device 810.

The pneumatic convective device 810 can be similar to pneumatic convective device 118 except that pneumatic convective device 810 is a single sheet 802. For example, the sheet 802 can be attached directly to the inner surface 122 of clinical garment 116. The sheet 802 can be sealed to the inner surface 122 along perimeter seal 816 to form a cavity. In at least one embodiment, the sheet 802 can include an edge portion 812 that is proximate to the neck opening 102.

In at least one embodiment, the warming device 800 can also include a secondary portion 818. The secondary portion 818 can be a separate layer of material or can be integrally formed with the clinical garment 116. The secondary portion 818 can extend beyond the hemline 108 such that the edge portion 804 covers a portion of the legs of a patient in a deployed state. In at least one embodiment, the secondary portion 818 can be a portion of the sheet 802 that is folded over and sealed along the edges of pneumatic convective device 810 to form a portion of the perimeter seal 816.

The sheet 802 can be sealed to the clinical garment 116 along perimeter seal 816. In at least one embodiment, the sheet 802 can formed of an air-permeable layer and the clinical garment 116 can be treated to be air-impermeable so that warmed air can be applied to a patient. The edge portion 804 can extend past the hemline 108 (which is aligned with axis 806). The secondary portion 818 can be at least partially defined by a dimension 808 from the axis 806 to the edge portion 804. In at least one embodiment, the dimension 808 is at least 15 cm (6 inches), at least 20 cm (8 inches), at least 25 cm (10 inches), or at least 30 cm (12 inches).

FIG. 9 illustrates a side cross-sectional view of the warming device 800. The pneumatic convective device 810 includes at least one sheet, e.g., sheet 802. The sheet 802 can be sealed to the inner surface 122 along a perimeter seal 816 to form a cavity 906. In at least one embodiment, the pneumatic convective device 810 can also include a secondary portion 818 that extends beyond the hemline 108. The secondary portion 818 can be a separate sheet that is attached to a portion of the inner surface 122 via a fastening device 904. In at least one embodiment, the fastening device 904 can be positioned proximate the hemline 108. For example, the fastening device 904 does not extend beyond position 902. In at least one embodiment, edge portion 812 can be attached to the inner surface 122 at position 910. In at least one embodiment, the second portion edge 912 can extend at some position between position 902 and position 910.

As shown, the secondary portion 818 can enable the sheet 802 to extend beyond the hemline 108 to cover a portion of the patient's legs and using less material. The cavity 906 formed by inner surface 122, sheet 802, and secondary portion 818 can also be inflatable via an inlet 908 which can be configured according to earlier embodiments.

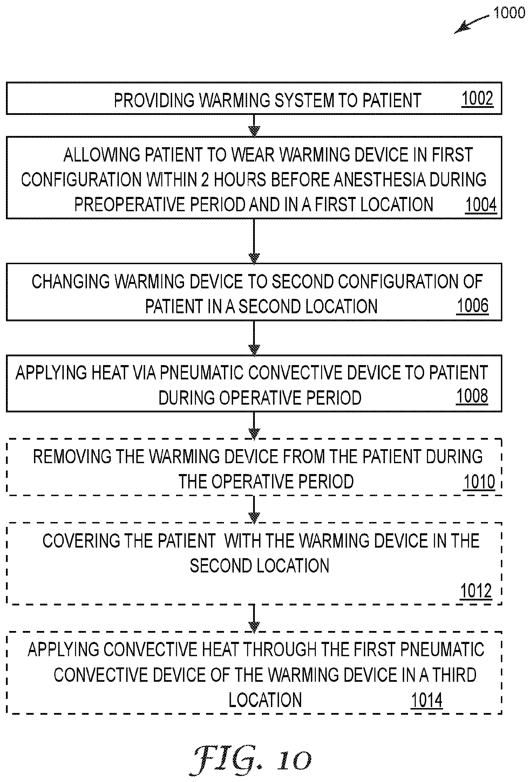

FIG. 10 illustrates a flowchart of a method 1000 of applying therapeutic warming to a patient.

The method 1000 can begin at block 1002. In block 1002, the warming system described herein can be provided to the patient. A manufacturer can provide one or more of the components of the warming system to the clinician who can further provide it to the patient according to the instructions of the manufacturer. In block 1004, the clinician can allow the patient to wear the warming device that can include at least a first pneumatic convective device. The patient can wear the warming device during a preoperative period of a perioperative period. For example, the patient can preferably wear the warming device within 2 hours, within 1 hour, or within 30 minutes before anesthesia.

The warming device can help to pre-warm the patient before the anesthesia sufficient to maintain normothermia during anesthesia. Optionally, block 1004 can also include applying heat to the patient via a first pneumatic convective device that is fixedly attached to the warming device. The clinician can activate an active convective heat modality for the warming device. The heat application during block 1004 can result in pre-warming prior to anesthesia. In at least one embodiment, the warming device can contain a passive modality for pre-warming the patient and reducing heat losses. For example, the warming device can also have an insulative material that can reduce heat loss by the patient.

In at least one embodiment, the clinician can place the warming device onto the patient in a first location. For example, the first location can be dependent on hospital configurations but the first location can be a separate surgical waiting area, a patient recovery room, or preoperative bay. Preferably, the first location can have a power outlet.

In block 1006, the clinician can change the warming device from a first configuration to a second configuration before anesthesia in a second location. In at least one embodiment, the second location is an operating theatre, or operating room where the surgery is taking place. In at least one embodiment, the first location and the second location are the same. For example, pre-warming of the second configuration can be related to a feature of the warming device. For example, the second configuration can be an opened pocket, a removable pneumatic convective device, or a deployment of a pneumatic convective device.

For example, in the current configuration, the first configuration can be in the worn configuration as a patient wears the clinical garment. This may include fastening the fastening devices of the clinical garment In the second configuration, the clinician can remove a pneumatic convective device that is releasably attached (i.e., recloseable fastener) to the surface of the clinical garment or an integrated pneumatic convective device thereon. The pneumatic convective device can be fastened with a repositionable adhesive, which is effective to allow the repositioning of the adhesive article from the first application surface to the second application surface without leaving any visible residue of the pressure sensitive adhesive on the first application surface, the visible residue being any residue that is visible to the unaided eye of a human being.

The changing to the second configuration can also occur during the operative period of the perioperative period. In at least one embodiment, the operative period can begin within 10 minutes, within 8 minutes, or within 5 minutes before anesthesia is delivered and continue while the patient is anesthetized.

For example, a clinician or the patient can be instructed to convert the first pneumatic convective device from a folded state into a partially deployed state or a deployed state. As used herein, the folded state, partially deployed state, or deployed state can refer to either the first or second configurations. For example, the folded state can be the first configuration and a partially deployed state or deployed can be the second configuration. In at least one embodiment, the deployed state can be the first configuration and the folded state can be the second configuration.

The patient can be ambulatory or stationary (e.g., in a sitting, standing, or supine position). For example, the patient can be stationary when undergoing prewarming in a supine position prior to the pneumatic convective device being in the deployed state. In at least one embodiment, the patient can be sitting while waiting for the surgery. The pneumatic convective device can cover at least a portion of the torso of the patient while in the folded state. In at least one embodiment, warmed air can be provided to the patient while in the folded state.

To improve the thermal energy delivered to the patient, the pneumatic surface area can be increased by converting the pneumatic convective device from a folded state to a partially deployed state. The partially deployed state can have a larger pneumatic surface area than the folded state. In at least one embodiment, the partially deployed state can also enable the patient to be ambulatory.

The pneumatic surface area can also be increased by converting the pneumatic convective device from a folded state to a deployed state. The deployed state can be at least 90 percent or 100 percent of available pneumatic surface area of the pneumatic convective device.

A clinician can grab a portion of the pneumatic convective device (e.g., a tab element) and, with a pull force away from the clinical garment, extend the pneumatic convective device to a partially deployed state or a deployed state.

In at least one embodiment, the partially deployed state can correspond to 40 to 70 percent, or 50 to 70 percent of the available pneumatic surface area of the pneumatic convective device.

In at least one embodiment, the deployed state can have a pneumatic surface area that is at least 25 percent, at least 30 percent, at least 40 percent, at least 45 percent, at least 75 percent, at least 80 percent, at least 85 percent, at least 90 percent, at least 95 percent, at least 100 percent, or at least 105 percent of the inner surface area of the clinical garment (including sleeves). In at least one embodiment, the partially deployed state can be no greater than 75 percent, no greater than 70 percent, no greater than 65 percent, no greater than 60 percent, no greater than 55 percent, no greater than 50 percent, or no greater than 45 percent but at a minimum of least 40 percent of the inner surface area of the clinical garment (including sleeves).

In at least one embodiment, the pneumatic convective device in the deployed state can be configured cover 80 to 100 percent of the patient, preferably 90 to 100 percent.

In block 1008, the clinician can apply heat to the patient during the operative period. The total amount of heat is dependent on the clinician preferences, however, the convective warming unit can be capable to output at least 300 watts, at least 350 watts, or at least 400 watts at a hose end. In at least one embodiment, the power consumption of the convective warming unit can be at least 1400 watts. In at least one embodiment, the convective warming unit is configured to output at least 0.425 m³/min (15 cfm), at least 0.708 m³/min (25 cfm), at least 0.991 m³/min (35 cfm), or at least 1.27 m³/min (45 cfm) to aid in heat transfer. The heat applied can be sufficient to maintain normothermia of a patient. In at least one embodiment, the heat can be applied through the warming device or from a separate pneumatic convective device that is attached thereon.

Block 1010 to block 1014 can be optional. In block 1010, the clinician can remove at least a portion the warming device from the patient during the operative period. For example, during a surgical procedure, the warming device can interfere with some surgical procedures (such as an abdominal incision). Thus, to provide warming, the warming device can be removed to expose a surgical treatment area, and optionally apply the heat via a separate pneumatic convective device that is part of the warming system. The warming device can be set aside for later use.

In at least one embodiment, in the deployed state, the pneumatic convective device can be folded over the hemline of the clinical garment and an end of the pneumatic convective device can be releasably attached to the outer surface of the clinical garment. This position can enable the clinician to operate on the patient or allow the patient to become ambulatory in a post-operative environment without danger of tripping.

In block 1012, the clinician can retrieve the warming device and cover the patient with the warming device. For example, after the surgical procedure is over, but while the patient is anesthetized, the clinician can cover the patient for modesty in the second location. After the patient is covered, then the patient can be transported to a third location. The third location can be a post-operative recovery bay (i.e., PACU) or a patient room. In at least one embodiment, the separate pneumatic convective device can be discarded.

In block 1014, the clinician can apply heat to the patient through the first pneumatic convective device attached to the warming device. The heat can be such that the patient is comfort warmed during the post-operative period of the perioperative period. The post-operative period can generally be in the third location. In at least one embodiment, the heat can be for comfort warming during block 1014. For example, the heat can be no greater than 500 watts, no greater than 400 watts, or no greater than 200 watts. The temperature at the hose-end, prior to the air entering the pneumatic convective device, may range from ambient to up to 46° C, but preferably at least 37 degrees C. The airflow at the hose-end, prior to the air entering the pneumatic convective device, may be between 0.142 - 0.425 m³/min (5-15 CFM) or even 0.850 - 1.42 m³/min (30 - 50 CFM). The pressure inside the pneumatic convective device may range of 0.13 to 3.0 cm H₂O (0.05 to 1.2 inches H₂O).

## Claims

1. A warming device (100, 800) comprising:
a clinical garment (116) comprising:
a body portion adapted to cover a portion of a patient, an inner surface (122) for facing the patient, and an outer surface (120) for facing away from the patient, wherein the inner surface has an inner surface area, wherein the body portion comprises a hemline (108) and forms a longitudinal axis (138), and
sleeves (112) sized and positioned for receiving a patient's arms;
a first pneumatic convective device (118, 810) proximate the inner surface and opposite from the outer surface, wherein the first pneumatic convective device has a first end (134) proximate the sleeves and a second end (136) oriented along the longitudinal axis of the clinical garment, wherein the first pneumatic convective device comprises an air-permeable layer having a pneumatic surface area, the pneumatic surface area is at least 25% of the inner surface area including the sleeves in a deployed state;
a first inlet (204, 908) for admitting a stream of pressurized, warmed air into the first pneumatic convective device, wherein the first inlet is on a side corresponding to the outer surface; and
wherein the second end of the first pneumatic convective device extends past the hemline when in the deployed state, and, when the first pneumatic convective device is in a folded state, the second end extends no greater than 7.6 cm (3 inches) past the hemline, and **characterized in that**
the pneumatic convective device (118) has a plurality of fold sections (314, 404, 406) in the folded state, each fold section having a fold edge portion (304, 306) formed from creasing the pneumatic convective device (118), different fold sections being attached to other fold sections using releasable fastening devices (308, 310) disposed proximate to one of the fold edge portions.

2. The warming device of claim 1, wherein the first pneumatic convective device (118, 810) has a first internal volume when inflated in the folded state, and the first pneumatic convective device has a second internal volume when inflated in the deployed state, the first internal volume is less than the second internal volume.

3. The warming device of claim 2, wherein, when the first pneumatic convective device (118, 810) is in the folded state, the first pneumatic convective device comprises a first fold section (314) defined by a first fold edge portion (306) and at least one pneumatic edge portion (128).

4. The warming device of claim 3, wherein the first internal volume is defined by a pneumatic edge portion (128) and the first fold edge portion (306), where the second internal volume is defined by a plurality of pneumatic edge portions.

5. The warming device of claim 3, wherein, when in the folded state, the first pneumatic convective device (118, 810) further comprises a second fold section (406) defined by a second fold edge portion (304).

6. The warming device of claim 5, wherein, when in the folded state, the first pneumatic convective device (118, 810) further comprises a third fold section defined by the first fold edge portion, the second fold edge portion and at least one pneumatic edge portion.

7. The warming device of claim 3, wherein the first pneumatic convective device (118, 810) comprises a tab element (302) extending past a pneumatic edge portion (128).

8. The warming device of claim 3, wherein, when in a partially deployed state, the first pneumatic convective device (118, 810) has a third internal volume in between the first internal volume and the second internal volume.

9. The warming device of claim 1, wherein the first pneumatic convective device (118, 810) comprises a first sheet that is air-permeable and sealed to the clinical garment (116) except at an unsealed portion, wherein the first pneumatic convective device comprises a second sheet that is air-impermeable, the second sheet is attached to the first sheet of material at most of the unsealed portion.

10. The warming device of claim 9, wherein the second sheet is attached to the inner surface (122).

11. The warming device of claim 9, wherein the inlet (204, 908) is proximate to a fold edge portion and disposed on the second sheet, when in the folded state, the inlet does not extend past the hemline (108), and when in the deployed state, the inlet extends past the hemline.

12. The warming device of any of the preceding claims, further comprising:
a second pneumatic convective device (142) fixedly attached to the inner surface (122), wherein the first pneumatic convective device is releasably attached to the second pneumatic convective device. wherein the second pneumatic convective device comprises a second inlet for admitting a stream of pressurized, warmed air into the second pneumatic convective device.

13. A warming system, comprising:
a warming device of any of claims 1 to 12; and
a second pneumatic convective device (142).

14. The warming system of claim 13, further comprising a convective warming unit configured to provide a stream of pressurized, warmed air to the inlet configurated to have a temperature of at least 37 degrees C and having an airflow rate of at least 0.850 m³/min (30 cubic feet per minute) at a hose end.

## Patentansprüche

1. Eine Wärmevorrichtung (100, 800), aufweisend:
ein klinisches Kleidungsstück (116), aufweisend:
einen Körperabschnitt, der ausgelegt ist, um einen Abschnitt eines Patienten zu bedecken, eine Innenoberfläche (122), die dem Patienten zugewandt ist, und eine Außenoberfläche (120), die von dem Patienten abgewandt ist, wobei die Innenoberfläche einen Innenoberflächenbereich aufweist, wobei der Körperabschnitt einen Saum (108) aufweist und eine Längsachse (138) bildet, und
Ärmel (112), die derart dimensioniert und positioniert sind, dass sie die Arme eines Patienten aufnehmen;
eine erste pneumatische Konvektionsvorrichtung (118, 810) nahe der Innenoberfläche und gegenüber der Außenoberfläche, wobei die erste pneumatische Konvektionsvorrichtung ein erstes Ende (134) nahe den Ärmeln und ein zweites Ende (136) aufweist, das entlang der Längsachse des klinischen Kleidungsstücks ausgerichtet ist, wobei die erste pneumatische Konvektionsvorrichtung eine luftdurchlässige Schicht aufweist, die einen pneumatischen Oberflächenbereich aufweist, wobei der pneumatische Oberflächenbereich mindestens 25 % des Innenoberflächenbereichs einschließlich der Ärmel in einem entfalteten Zustand beträgt;
einen ersten Einlass (204, 908) zum Einlassen eines Stroms von unter Druck stehender, erwärmter Luft in die erste pneumatische Konvektionsvorrichtung, wobei der erste Einlass auf einer Seite ist, die der Außenoberfläche entspricht; und
wobei das zweite Ende der ersten pneumatischen Konvektionsvorrichtung über den Saum hinausragt, wenn sie sich in dem entfalteten Zustand befindet, und, wenn sich die erste pneumatische Konvektionsvorrichtung in einem gefalteten Zustand befindet, das zweite Ende nicht mehr als 7,6 cm (3 Zoll) über den Saum hinausragt, und **dadurch gekennzeichnet, dass**
die pneumatische Konvektionsvorrichtung (118) mehrere Faltenabschnitte (314, 404, 406) in dem gefalteten Zustand aufweist, wobei jeder Faltenabschnitt einen Faltenkantenabschnitt (304, 306) aufweist, der durch Falten der pneumatischen Konvektionsvorrichtung (118) gebildet wird, wobei verschiedene Faltenabschnitte unter Verwendung von lösbaren Befestigungsvorrichtungen (308, 310), die in der Nähe eines der Faltenkantenbereiche angeordnet sind, an anderen Faltenabschnitten befestigt sind.

2. Die Wärmevorrichtung nach Anspruch 1, wobei die erste pneumatische Konvektionsvorrichtung (118, 810) ein erstes Innenvolumen aufweist, wenn sie in dem gefalteten Zustand aufgeblasen ist, und die erste pneumatische Konvektionsvorrichtung ein zweites Innenvolumen aufweist, wenn sie in dem entfalteten Zustand aufgeblasen ist, wobei das erste Innenvolumen kleiner ist als das zweite Innenvolumen.

3. Die Wärmevorrichtung nach Anspruch 2, wobei, wenn sich die erste pneumatische Konvektionsvorrichtung (118, 810) in dem gefalteten Zustand befindet, die erste pneumatische Konvektionsvorrichtung einen ersten Faltenabschnitt (314) aufweist, der durch einen ersten Faltenkantenabschnitt (306) und mindestens einen pneumatischen Kantenabschnitt (128) definiert ist.

4. Die Wärmevorrichtung nach Anspruch 3, wobei das erste Innenvolumen durch einen pneumatischen Kantenabschnitt (128) und den ersten Faltenkantenabschnitt (306) definiert ist, wobei das zweite Innenvolumen durch mehrere pneumatische Kantenabschnitte definiert ist.

5. Die Wärmevorrichtung nach Anspruch 3, wobei, wenn sie sich in dem gefalteten Zustand befindet, die erste pneumatische Konvektionsvorrichtung (118, 810) ferner einen zweiten Faltenabschnitt (406) aufweist, der durch einen zweiten Faltenkantenabschnitt (304) definiert ist.

6. Die Wärmevorrichtung nach Anspruch 5, wobei, wenn sie sich in dem gefalteten Zustand befindet, die erste pneumatische Konvektionsvorrichtung (118, 810) ferner einen dritten Faltenabschnitt aufweist, der durch den ersten Faltenkantenabschnitt, den zweiten Faltenkantenabschnitt und mindestens einen pneumatischen Kantenabschnitt definiert ist.

7. Die Wärmevorrichtung nach Anspruch 3, wobei die erste pneumatische Konvektionsvorrichtung (118, 810) ein Laschenlement (302) aufweist, das sich über einen pneumatischen Kantenabschnitt (128) hinaus erstreckt.

8. Die Wärmevorrichtung nach Anspruch 3, wobei, wenn sie sich in einem teilweise entfalteten Zustand befindet, die erste pneumatische Konvektionsvorrichtung (118, 810) ein drittes Innenvolumen zwischen dem ersten Innenvolumen und dem zweiten Innenvolumen aufweist.

9. Die Wärmevorrichtung nach Anspruch 1, wobei die erste pneumatische Konvektionsvorrichtung (118, 810) eine erste Lage aufweist, die luftdurchlässig ist und an das klinische Kleidungsstück (116) abgedichtet ist, außer an einem nicht abgedichteten Abschnitt, wobei die erste pneumatische Konvektionsvorrichtung eine zweite Lage aufweist, die luftundurchlässig ist, wobei die zweite Lage an der ersten Lage aus Material an dem größten Teil des nicht abgedichteten Abschnitts befestigt ist.

10. Die Wärmevorrichtung nach Anspruch 9, wobei die zweite Lage an der Innenoberfläche (122) befestigt ist.

11. Die Wärmevorrichtung nach Anspruch 9, wobei sich der Einlass (204, 908) in der Nähe eines Faltenkantenabschnitts befindet und auf der zweiten Lage angeordnet ist, wobei sich der Einlass in dem gefalteten Zustand nicht über den Saum (108) hinaus erstreckt und sich der Einlass in dem entfalteten Zustand über den Saum hinaus erstreckt.

12. Die Wärmevorrichtung nach einem der vorstehenden Ansprüche, ferner umfassend:
eine zweite pneumatische Konvektionsvorrichtung (142), die fest an der Innenoberfläche (122) befestigt ist, wobei die erste pneumatische Konvektionsvorrichtung lösbar an der zweiten pneumatischen Konvektionsvorrichtung befestigt ist, wobei die zweite pneumatische Konvektionsvorrichtung einen zweiten Einlass zum Einlassen eines Stroms von unter Druck stehender, erwärmter Luft in die zweite pneumatische Konvektionsvorrichtung aufweist.

13. Ein Wärmesystem, aufweisend:
eine Wärmevorrichtung nach einem der Ansprüche 1 bis 12; und
eine zweite pneumatische Konvektionsvorrichtung (142).

14. Das Wärmesystem nach Anspruch 13, ferner aufweisend eine Konvektionswärmeeinheit, die konfiguriert ist, um einen Strom von unter Druck stehender, erwärmter Luft zu dem Einlass bereitzustellen, der konfiguriert ist, um eine Temperatur von mindestens 37 Grad C zu haben und eine Luftströmungsrate von mindestens 0,850 m³/min (30 Kubikfuß pro Minute) an einem Schlauchende aufzuweisen.

## Revendications

1. Dispositif de réchauffement (100, 800) comprenant :
un vêtement clinique (116), comprenant :
une partie de corps adaptée pour couvrir une partie d'un patient, une surface interne (122) destinée à être tournée vers le patient, et une surface externe (120) destinée à être tournée à l'écart du patient, dans lequel la surface interne a une zone de surface interne, dans lequel la partie de corps comprend un ourlet (108) et forme un axe longitudinal (138), et
des manches (112) dimensionnés et positionnés pour recevoir les bras d'un patient ;
un premier dispositif de convection pneumatique (118, 810) à proximité de la surface interne et à l'opposé de la surface externe, dans lequel le premier dispositif de convection pneumatique a une première extrémité (134) à proximité des manches et une seconde extrémité (136) orientée le long de l'axe longitudinal du vêtement clinique, dans lequel le premier dispositif de convection pneumatique comprend une couche perméable à l'air ayant une zone de surface pneumatique, la zone de surface pneumatique est d'au moins 25 % de la zone de surface interne comportant les manches dans un état déployé ;
une première entrée (204, 908) destinée à admettre un flux d'air réchauffé sous pression dans le premier dispositif de convection pneumatique, dans lequel la première entrée est sur un côté correspondant à la surface externe ; et
dans lequel la seconde extrémité du premier dispositif de convection pneumatique s'étend au-delà de l'ourlet lorsqu'il est dans l'état déployé, et, lorsque le premier dispositif de convection pneumatique est dans un état plié, la seconde extrémité ne s'étend pas plus de 7,6 cm (3 pouces) au-delà de l'ourlet, et **caractérisé en ce que**
le dispositif de convection pneumatique (118) a une pluralité de sections de pli (314, 404, 406) dans l'état plié, chaque section de pli ayant une partie de bord de pli (304, 306) formée à partir du rainurage du dispositif de convection pneumatique (118), différentes sections de pli étant fixées à d'autres sections de pli à l'aide de dispositifs d'attache amovibles (308, 310) disposés à proximité de l'une des parties de bord de pli.

2. Dispositif de réchauffement selon la revendication 1, dans lequel le premier dispositif de convection pneumatique (118, 810) a un premier volume interne lorsqu'il est gonflé dans l'état plié, et le premier dispositif de convection pneumatique a un second volume interne lorsqu'il est gonflé dans l'état déployé, le premier volume interne est inférieur au second volume interne.

3. Dispositif de réchauffement selon la revendication 2, dans lequel, lorsque le premier dispositif de convection pneumatique (118, 810) est dans l'état plié, le premier dispositif de convection pneumatique comprend une première section de pli (314) définie par une première partie de bord de pli (306) et au moins une partie de bord pneumatique (128).

4. Dispositif de réchauffement selon la revendication 3, dans lequel le premier volume interne est défini par une partie de bord pneumatique (128) et la première partie de bord de pli (306), où le second volume interne est défini par une pluralité de parties de bord pneumatiques.

5. Dispositif de réchauffement selon la revendication 3, dans lequel, dans l'état plié, le premier dispositif de convection pneumatique (118, 810) comprend en outre une deuxième section de pli (406) définie par une seconde partie de bord de pli (304).

6. Dispositif de réchauffement selon la revendication 5, dans lequel, dans l'état plié, le premier dispositif de convection pneumatique (118, 810) comprend en outre une troisième section de pli définie par la première partie de bord de pli, la seconde partie de bord de pli et au moins une partie de bord pneumatique.

7. Dispositif de réchauffement selon la revendication 3, dans lequel le premier dispositif de convection pneumatique (118, 810) comprend un élément de languette (302) s'étendant au-delà d'une partie de bord pneumatique (128).

8. Dispositif de réchauffement selon la revendication 3, dans lequel, lorsqu'il est dans un état partiellement déployé, le premier dispositif de convection pneumatique (118, 810) a un troisième volume interne entre le premier volume interne et le deuxième volume interne.

9. Dispositif de réchauffement selon la revendication 1, dans lequel le premier dispositif de convection pneumatique (118, 810) comprend une première feuille qui est perméable à l'air et scellée au vêtement clinique (116) sauf au niveau d'une partie non scellée, dans lequel le premier dispositif de convection pneumatique comprend une seconde feuille qui est imperméable à l'air, la seconde feuille est fixée à la première feuille de matériau au maximum de la partie non scellée.

10. Dispositif de réchauffement selon la revendication 9, dans lequel la seconde feuille est fixée à la surface interne (122).

11. Dispositif de réchauffement selon la revendication 9, dans lequel l'entrée (204, 908) est à proximité d'une partie de bord de pli et disposée sur la seconde feuille, lorsqu'elle est dans l'état plié, l'entrée ne dépasse pas l'ourlet (108), et lorsqu'elle est dans l'état déployé, l'entrée s'étend au-delà de l'ourlet.

12. Dispositif de réchauffement selon l'une quelconque des revendications précédentes, comprenant en outre :
un second dispositif de convection pneumatique (142) solidement fixé à la surface interne (122), dans lequel le premier dispositif de convection pneumatique est fixé de manière amovible au second dispositif de convection pneumatique, dans lequel le second dispositif de convection pneumatique comprend une seconde entrée destinée à admettre un flux d'air réchauffé sous pression dans le second dispositif de convection pneumatique.

13. Système de réchauffement, comprenant :
un dispositif de réchauffement selon l'une quelconque des revendications 1 à 12 ; et
un second dispositif de convection pneumatique (142).

14. Système de réchauffement selon la revendication 13, comprenant en outre une unité de réchauffement par convection configurée pour fournir un flux d'air réchauffé sous pression à l'entrée, configuré pour avoir une température d'au moins 37 degrés C et ayant un débit de flux d'air d'au moins 0,850 m³/min (30 pieds cubes par minute) au niveau d'une extrémité de tuyau.
